# EUROPEAN PATENT APPLICATION

(11) **EP 4 480 908 A1**
(43) Date of publication of application: **25.12.2024**
(21) Application number: 22930903.4
(22) Date of filing: 11.03.2022
(51) Int. Cl.: C01B 32/168, C07K 1/14

(54) **ADSORBENT AND METHOD FOR PRODUCING ADSORBENT**

(71) Applicant: Hoya Technosurgical Corporation, Tokyo 160-0004 (JP)
(72) Inventor: YOSHITAKE Tomohiko, Tokyo 160-0004 (JP)
(74) Representative: Diehl & Partner
(86) International application number: PCT/JP2022/010908
(87) International publication number: WO 2023/170922

(57) **Abstract**

Object: To provide an adsorbent having improved durability and improved adsorbability of a substance to be adsorbed, and a method for manufacturing the adsorbent.

Resolution means: An adsorbent of the present invention is to be packed in a column, and is composed of powder containing particles whose surface is made of hydroxyapatite or fluorapatite in which at least some of hydroxyl groups of the hydroxyapatite have been replaced with fluorine atoms, the powder being composed of porous bodies with pores, the powder having an average particle size of 10 µm or more and 90 µm or less, the powder having an average particle compressive strength of 7.4 MPa or more and 8.9 MPa or less, and a modal pore size in a pore size distribution of the pores of the powder being 32.0 nm or more and 60.0 nm or less.

## Description

### Technical Field

The present invention relates to an adsorbent having improved durability and improved adsorbability of a substance to be adsorbed, and a method for manufacturing the adsorbent.

### Background Art

In recent years, hydroxyapatite, because of its high biocompatibility and excellent safety, for example, has been widely used as a material for stationary layer of chromatography, i.e., an adsorbent, for use in purification and isolation of bio-based pharmaceuticals such as antibodies and vaccines.

Hydroxyapatite (HAP) used as a material for stationary layer of chromatography is manufactured, for example, as follows.

Specifically, a first liquid containing calcium hydroxide and a second liquid containing phosphoric acid are reacted under stirring to obtain primary particles of hydroxyapatite, a slurry containing the primary particles and agglomerates thereof is dried and granulated, thereby obtaining secondary particles (powder) of hydroxyapatite.

This powder (dried powder) is baked to obtain a sintered powder, and the green powder or sintered powder is charged into a column (adsorption apparatus), etc. so as to be used as a material for stationary layer (adsorbent) (see, for example, Patent Document 1).

However, when sintered powder is used as the adsorbent in an adsorption apparatus as described above, it was the fact that there was non-uniformity in the durability of the adsorbent, i.e., the sintered powder, and in the adsorbability of the biopharmaceuticals to be purified and isolated, i.e., the substance to be adsorbed. These non-uniformities cause problems that the uniform column performance of purifying and isolating the substance to be adsorbed cannot be imparted to an adsorption apparatus including the adsorbent, and that non-uniformity in the durability of the adsorbent occurs. The present inventors have found the presence of such problems.

### Citation List

### Patent Document

Patent Document 1: JP 2011-68539 A

### Summary of Invention

### Technical Problem

An object of the present invention is to provide an adsorbent having improved durability and improved adsorbability of a substance to be adsorbed, and a method for manufacturing the adsorbent.

### Solution to Problem

The object is achieved by the present invention described in (1) to (19) below.
(1) An adsorbent which is to be packed in a column, and is composed of powder containing particles whose surface is made of hydroxyapatite or fluorapatite in which at least some of hydroxyl groups of the hydroxyapatite have been replaced with fluorine atoms,
   the powder being composed of porous bodies with pores,
   the powder having an average particle size of 10 µm or more and 90 µm or less,
   the powder having an average particle compressive strength of 7.4 MPa or more and 8.9 MPa or less, and
   a modal pore size in a pore size distribution of the pores of the powder being 32.0 nm or more and 60.0 nm or less.

Accordingly, the sintered powder has improved durability and improved adsorbability to a substance to be adsorbed.

(2) The adsorbent recited in the above (1), wherein a specific surface area of the powder is 41 m²/g or more and 51 m²/g or less.

Accordingly, even if the isolation material (substance to be adsorbed) is a relatively large protein such as antibodies (antibody molecules), it can be said that sintered powder has the surface area necessary to adsorb a sufficient amount of the isolation material. Therefore, the sintered powder can have further improved adsorbability of the substance to be adsorbed.

(3) The adsorbent recited in the above (1), wherein a surface area of the powder per 1 mL of the column is 25.3 m²/mL or more and 36.0 m²/mL or less.

Accordingly, even if the isolation material (substance to be adsorbed) is a relatively large protein such as antibodies (antibody molecules), it can be said that sintered powder has the surface area necessary to adsorb a sufficient amount of the isolation material. Therefore, the sintered powder can have further improved adsorbability of the substance to be adsorbed.

(4) An adsorbent which is to be packed in a column, and is composed of sintered powder obtained by baking particles whose surface is made of hydroxyapatite or fluorapatite in which at least some of the hydroxyl groups of the hydroxyapatite have been replaced with fluorine atoms,
the sintered powder having an average particle size of 10 µm or more and 90 µm or less,
the sintered powder having a specific surface area of 41 m²/g or more and 51 m²/g or less, and
a modal pore size in a pore size distribution of pores of the sintered powder being 32.0 nm or more and 60.0 nm or less.

Accordingly, even relatively large proteins such as antibodies (antibody molecules) can be reliably adsorbed to the sintered powder by capturing them in the pores of the sintered powder as the isolation material. In addition, even if the isolation material (substance to be adsorbed) is a relatively large protein such as antibodies (antibody molecules), it can be said that sintered powder has the surface area necessary to adsorb a sufficient amount of the isolation material. Therefore, the sintered powder can have further improved adsorbability of the substance to be adsorbed.

(5) An adsorbent which is to be packed in a column, and is composed of powder containing particles whose surface is made of hydroxyapatite or fluorapatite in which at least some of hydroxyl groups of the hydroxyapatite have been replaced with fluorine atoms,
the powder having an average particle size of 10 µm or more and 90 µm or less,
a surface area of the powder per 1 mL of the column being 25.3 m²/mL or more and 36.0 m²/mL or less, and
a modal pore size in a pore size distribution of pores of the powder being 32.0 nm or more and 60.0 nm or less.

Accordingly, even relatively large proteins such as antibodies (antibody molecules) can be reliably adsorbed to the sintered powder by capturing them in the pores of the sintered powder as the isolation material. In addition, even if the isolation material (substance to be adsorbed) is a relatively large protein such as antibodies (antibody molecules), it can be said that sintered powder has the surface area necessary to adsorb a sufficient amount of the isolation material. Therefore, the sintered powder can have further improved adsorbability of the substance to be adsorbed.

(6) The adsorbent recited in the above (1), (3) or (5), wherein a frequency of the modal pore size is 21.0% or more and 60.0% or less.

Accordingly, even relatively large proteins such as antibodies (antibody molecules) can be reliably adsorbed to the sintered powder by capturing them in the pores of the sintered powder as the isolation material.

(7) The adsorbent recited in the above (1), (3) or (5), wherein a half-width of a mountain-shaped waveform around the modal pore size in the pore size distribution of the pores is 1.0 nm or more and 10.0 nm or less.

Accordingly, even relatively large proteins such as antibodies (antibody molecules) can be reliably adsorbed to the sintered powder by capturing them in the pores of the sintered powder as the isolation material.

(8) The adsorbent recited in the above (1), (3) or (5), wherein an average pore size of the pores is 37.0 nm or more and 75.0 nm or less.

Accordingly, even relatively large proteins such as antibodies (antibody molecules) can be reliably adsorbed to the sintered powder by capturing them in the pores of the sintered powder as the isolation material.

(9) The adsorbent recited in any one of the above (1) to (5), wherein the powder is a sintered powder baked at 480°C or higher and 645°C or lower.

Accordingly, the resultant sintered powder can be relatively easily made to have an average particle compressive strength of 7.4 MPa or more and 8.9 MPa or less, and a modal pore size in the pore size distribution of the pores of the sintered powder of 32.0 nm or more and 60.0 nm or less. Therefore, the sintered powder has improved durability and improved adsorbability to a substance to be adsorbed.

(10) A method for manufacturing an adsorbent comprising:
a first step of preparing particles whose surface is made of hydroxyapatite or fluorapatite in which at least some of the hydroxyl groups of the hydroxyapatite have been replaced with fluorine atoms; and
a second step of baking the particles prepared in the first step at a temperature T in the range of 480°C or more and 645°C or less to obtain the sintered powder of the particles;
the sintered powder obtained in the second step having an average particle size of 10 µm or more and 90 µm or less, an average particle compressive strength of 7.4 MPa or more and 8.9 MPa or less, and a pore size of 32.0 nm or more and 60.0 nm or less.

Accordingly, the resultant sintered powder has improved durability and improved adsorbability to a substance to be adsorbed.

(11) A method for manufacturing an adsorbent comprising:
a first step of preparing particles whose surface is made of hydroxyapatite or fluorapatite in which at least some of the hydroxyl groups of the hydroxyapatite have been replaced with fluorine atoms; and
a second step of baking the particles prepared in the first step at a temperature T in the range of 480°C or more and 645°C or less to obtain the sintered powder of the particles;
the sintered powder obtained in the second step having an average particle size of 10 µm or more and 90 µm or less, a specific surface area of 41 m²/g or more and 51 m²/g or less, and a pore size of 32.0 nm or more and 60.0 nm or less.

Accordingly, the resultant sintered powder has improved durability and improved adsorbability to a substance to be adsorbed.

(12) A method for manufacturing an adsorbent comprising:
a first step of preparing particles whose surface is composed of hydroxyapatite or fluorapatite in which at least some of the hydroxyl groups of the hydroxyapatite have been replaced with fluorine atoms; and
a second step of baking the particles prepared in the first step at a temperature T in the range of 480°C or more and 645°C or less to obtain the sintered powder of the particles;
the sintered powder obtained in the second step having an average particle size of 10 µm or more and 90 µm or less,
a surface area of the sintered powder per 1 mL of a column when the sintered powder is packed in the column being 25.3 m²/mL or more and 36.0 m²/mL or less, and
a pore size is 32.0 nm or more and 60.0 nm or less.
Accordingly, the resultant sintered powder has improved durability and improved adsorbability to a substance to be adsorbed.

(13) An adsorbent which is to be packed in a column, and is composed of powder containing particles whose surface is made of hydroxyapatite or fluorapatite in which at least some of hydroxyl groups of the hydroxyapatite have been replaced with fluorine atoms, wherein
when a sample liquid is prepared by dissolving γ-Globulins from bovine blood, as a substance to be adsorbed, in 10 mM sodium phosphate buffer (pH 6.5) so that the concentration is 1.0 mg/mL, and the prepared sample liquid is supplied into the column (ϕ6.0×35 mm) at flow rates of 150 cm/h and 300 cm/h, and when for each flow rate, an absorbance value X (280 nm) of the sample liquid to be supplied into the column and an absorbance value Y (wavelength: 280 nm) of an eluate eluted from the column are measured, and adsorption amounts A (mg/mL) and D (mg/mL) of the substance to be adsorbed to the powder are calculated based on the amount of the sample liquid sent until the absorbance value Y (wavelength: 280 nm) becomes 10% of the absorbance value X (wavelength: 280 nm), the powder satisfies at least one of A≥31 mg/mL or more and 0.60≤D/A≤1.00, and 0.60≤D/A≤0.92.

Accordingly, it can be said that the adsorption amount of antibodies to the sintered powder is independent of the flow rate supplied into the column of the test liquid containing the antibodies. Therefore, during isolation and purification of antibodies using the adsorption apparatus including the sintered powder as the adsorbent, when supplying the sample liquid containing antibodies to the adsorption apparatus, even if the sample liquid cannot be supplied at a constant rate and thus non-uniformity occurs in the initial and final stages, the antibodies can be reliably adsorbed onto the sintered powder at a constant range of adsorption amount.

(14) An adsorbent which is to be packed in a column, and is composed of powder obtained by baking particles whose surface is made of hydroxyapatite or fluorapatite in which at least some of the hydroxyl groups of the hydroxyapatite have been replaced with fluorine atoms, wherein
when a sample liquid is prepared by dissolving γ-Globulins from bovine blood, as a substance to be adsorbed, in 10 mM sodium phosphate buffer (pH 6.5) so that the concentration is 1.0 mg/mL, and the prepared sample liquid is supplied into the column (ϕ6.0×35 mm) at flow rates of 200 cm/h and 300 cm/h, and when for each flow rate, an absorbance value X (280 nm) of the sample liquid to be supplied into the column and an absorbance value Y (wavelength: 280 nm) of an eluate eluted from the column are measured, and adsorption amounts B (mg/mL) and D (mg/mL) of the substance to be adsorbed to the powder are calculated based on the amount of the sample liquid sent until the absorbance value Y (wavelength: 280 nm) becomes 10% of the absorbance value X (wavelength: 280 nm), the powder satisfies at least one of B≥30 mg/mL or more and 0.75≤DB≤1.00, and 0.75≤DB≤0.96.

Accordingly, it can be said that the adsorption amount of antibodies to the sintered powder is independent of the flow rate supplied into the column of the test liquid containing the antibodies. Therefore, during isolation and purification of antibodies using the adsorption apparatus including the sintered powder as the adsorbent, when supplying the sample liquid containing antibodies to the adsorption apparatus, even if the sample liquid cannot be supplied at a constant rate and thus non-uniformity occurs in the initial and final stages, the antibodies can be reliably adsorbed onto the sintered powder at a constant range of adsorption amount.

(15) An adsorbent which is to be packed in a column, and is composed of powder obtained by baking particles whose surface is made of hydroxyapatite or fluorapatite in which at least some of the hydroxyl groups of the hydroxyapatite have been replaced with fluorine atoms, wherein
when a sample liquid is prepared by dissolving γ-Globulins from bovine blood, as a substance to be adsorbed, in 10 mM sodium phosphate buffer (pH 6.5) so that the concentration is 1.0 mg/mL, and the prepared sample liquid is supplied into the column (ϕ6.0×35 mm) at flow rates of 200 cm/h and 250 cm/h, and when for each flow rate, an absorbance value X (280 nm) of the sample liquid to be supplied into the column and an absorbance value Y (wavelength: 280 nm) of an eluate eluted from the column are measured, and adsorption amounts B (mg/mL) and C (mg/mL) of the substance to be adsorbed to the powder are calculated based on the amount of the sample liquid sent until the absorbance value Y (wavelength: 280 nm) becomes 10% of the absorbance value X (wavelength: 280 nm), the powder satisfies at least one of B≥30 mg/mL or more and 0.89≤CB≤1.00, and 0.89≤CB≤0.96.

Accordingly, it can be said that the adsorption amount of antibodies to the sintered powder is independent of the flow rate supplied into the column of the test liquid containing the antibodies. Therefore, during isolation and purification of antibodies using the adsorption apparatus including the sintered powder as the adsorbent, when supplying the sample liquid containing antibodies to the adsorption apparatus, even if the sample liquid cannot be supplied at a constant rate and thus non-uniformity occurs in the initial and final stages, the antibodies can be reliably adsorbed onto the sintered powder at a constant range of adsorption amount.

(16) The adsorbent recited in any one of the above (13) to (15), wherein
when a durability test of the powder is conducted in which the column (ϕ4.0×100 mm) which is dry-packed with the powder as the adsorbent, and each buffer is sent through the column in the order of step numbers shown in Table 1, and regarding that steps 1 to 5 shown in Table 1 are counted as one cycle, the cycle is repeated until the pressure inside the column exceeds 200 psi, the number of cycles repeated is 60 or more.

**[Table 1]**

| Step No. | Buffer Type | | Supply Amount | | Supply Time |
|---|---|---|---|---|---|
| | | | (CV) | (mL) | (hr) |
| 1 | 10 mM NaP pH 6.5 | | 15 | 18.8 | 0.63 |
| 2 | 10 mM NaP + 1 M NaCl | pH 6.5 | 10 | 12.6 | 0.42 |
| 3 | 0.4 M NaP pH 6.5 | | 10 | 12.6 | 0.42 |
| 4 | 3 M NaOH | | 10 | 12.6 | 0.42 |
| 5 | 0.4 M NaP pH 6.5 | | 10 | 12.6 | 0.42 |
| | SUM | | 55 | 69.1 | 2.30 |
| Flow rate (mL/min) | | 0.50 | | | |
| Linear velocity (cm/hr) | | 239 | | | |

As described above, the sintered powder has improved durability because it can perform 60 or more cycles in the durability test.

(17) The adsorbent recited in any one of the above (13) to (15), wherein the substance to be adsorbed is antibodies.

In the adsorbent of the present invention, antibodies are preferably selected as the substance to be adsorbed.

### Advantageous Effects of Invention

The adsorbent of the present invention has improved durability and improved adsorbability to a substance to be adsorbed. In addition, the adsorbent manufacturing method of the present invention is capable of manufacturing the adsorbent.

### Brief Description of Drawings

FIG. 1 is a vertical cross-sectional view illustrating an example of an adsorption apparatus including the adsorbent of the present invention.

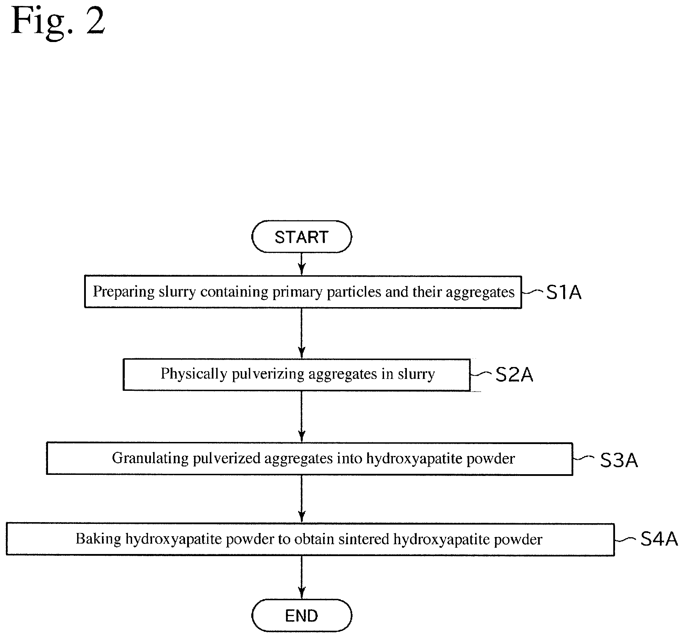

FIG. 2 is a flowchart illustrating a method of manufacturing an adsorbent of the present invention.
FIG. 3 is a graph showing a relationship between the adsorption amounts A to D of bIgG to sintered hydroxyapatite powder in each Example and Comparative Example and the flow rates of the sample liquid.

### Description of Embodiments

An adsorbent and a method of manufacturing an adsorbent according to the present invention will be described below in detail based on preferred embodiments illustrated in the accompanying drawings.

First, before describing the adsorbent and its manufacturing method of the present invention, an example of an adsorption apparatus (separation apparatus) including the adsorbent of the present invention will be described.

### <Adsorption apparatus>

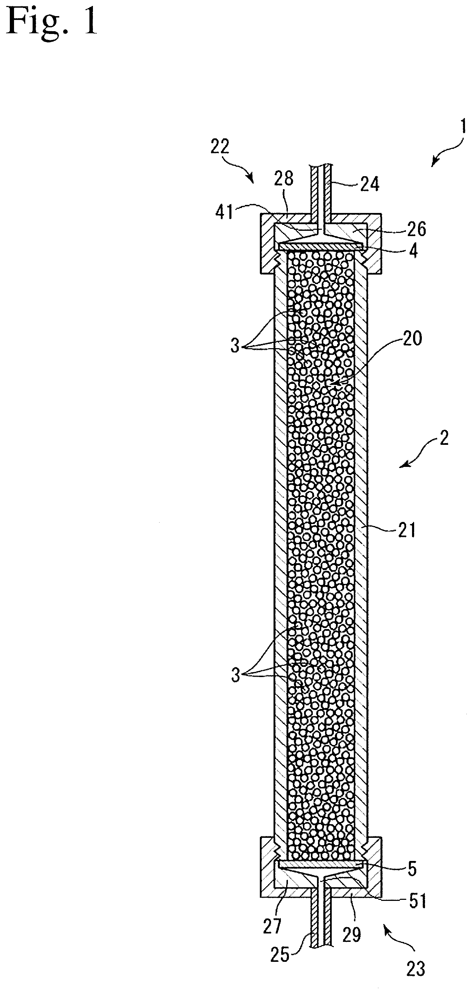

FIG. 1 is a vertical cross-sectional view illustrating an example of an adsorption apparatus including the adsorbent of the present invention. Note that, in the following description, the upper side in FIG. 1 is referred to as "inflow side" and the lower side is referred to as "outflow side".

Here, the inflow side refers to a side where, for example, a liquid such as a sample liquid (a liquid containing a substance to be adsorbed) or an eluate (such as a phosphoric acid-based buffer solution or water) is supplied into the adsorption apparatus, whereas the outflow side refers to a side opposite the inflow side, that is, a side where the liquid flows out of the adsorption apparatus, when the substance to be adsorbed, as an isolation material such as a biopharmaceutical, i.e., a protein to be separated is separated (purified).

An adsorption apparatus 1, as illustrated in FIG. 1, which separates (purifies) an isolation material (substance to be adsorbed) such as a protein from a sample liquid includes a column 2, a granular adsorbent 3 (filler), and two filter members 4 and 5.

The column 2 includes a column main body 21 and a cover portion 22 (first port) and a cover portion 23 (second port) attached to an inflow side end and an outflow side end of the column main body 21, respectively.

The column main body 21 is composed of a cylindrical member, for example. Examples of constituent materials of each component (each member) constituting the column 2 including the column main body 21 include various glass materials, various resin materials, various metal materials, and various ceramic materials.

In the column main body 21, the cover portions 22 and 23 are attached to the inflow side end and the outflow side end thereof, respectively, in a state in which the filter members 4 and 5 are disposed so as to close an inflow side opening and an outflow side opening of the column main body 21, respectively.

In the column 2 having such a configuration, the column main body 21 and the filter members 4 and 5 define an adsorbent filling space 20. Additionally, the column main body 21 and the cover portions 22 and 23 ensure liquid tightness of the adsorbent filling space 20. The adsorbent 3 is charged into at least part (substantially a full volume, in the present embodiment) of the adsorbent filling space 20.

An inner diameter of the adsorbent filling space 20 (column inner diameter) is set appropriately in accordance with a volume of a sample liquid, and is not particularly limited, but is, for example, preferably approximately 3.0 mm or more and 70.0 mm or less, and more preferably approximately 5.0 mm or more and 50.0 mm or less. In other words, a lower limit value of the inner diameter of the adsorbent filling space 20 is preferably 3.0 mm or more, and more preferably 5.0 mm or more, and its upper limit value is preferably 70.0 mm or less, and more preferably 50.0 mm or less. In addition, a length of the adsorbent filling space 20 (column length) is, for example, preferably approximately 10.0 mm or more and 300.0 mm or less, and more preferably approximately 20.0 mm or more and 200.0 mm or less. In other words, a lower limit value of the length of the adsorbent filling space 20 (column length) is preferably 10.0 mm or more, and more preferably 20.0 mm or more, and its upper limit value is preferably 300.0 mm or less, and more preferably 200.0 mm or less. By using the column 2 having such dimensions (inner diameter and length) of the adsorbent filling space 20 to isolate the isolation material contained in the sample liquid, the isolation material can be purified with excellent precision.

By setting the dimensions of the adsorbent filling space 20 to the values as described above and setting the dimensions of the adsorbent 3 described later to values as will be described below, it is possible to selectively isolate (purify) the target material to be isolated from the sample liquid, that is, to reliably separate an isolation material (substance to be adsorbed) such as a protein and contamination substances other than the isolation material contained in the sample liquid from each other.

Note that the isolation material to be isolated (purified) using the adsorbent 3 is not limited to proteins such as albumin, acidic proteins such as antibodies (antibody molecules), and basic proteins, and that examples thereof include negatively charged substances such as acidic amino acids, DNA, RNA, and negatively charged liposomes; and positively charged substances such as basic amino acids, positively charged cholesterol, and positively charged liposomes, in which proteins are preferably selected. That is, various substances including bio-based pharmaceuticals such as antibodies and vaccines can be purified and isolated, as substances to be adsorbed, using the adsorbent 3.

Further, the cover portions 22 and 23 are attached to the column main body 21 to ensure liquid tightness therebetween.

The cover portion 22 (first port) and the cover portion 23 (second port) include a cap 28 and a cap 29, an inlet pipe 24 (first flow path) and an outlet pipe 25 (second flow path), and a lid 26 and a lid 27, respectively.

The caps 28 and 29 are attached by screwing to the inflow side end (one end) and the outflow side end (the other end) of the column main body 21, with the inflow side end of the column main body 21 being located vertically above and the outflow side end being located vertically below. The caps 28 and 29 and the column main body 21 ensure liquid tightness therebetween.

The inlet pipe 24 and the outlet pipe 25 are composed of a tube body through which the liquid flows, and are adhered (fixed) liquid-tightly to substantially centers of the caps 28 and 29, respectively. The lids 26 and 27 include flow paths 41 and 51, disposed between the filter members 4 and 5 and the caps 28 and 29 and communicating with the inlet pipe 24 and the outlet pipe 25. The liquid is supplied to the adsorbent 3 via the inlet pipe 24, the lid 26, and the filter member 4. The sample liquid supplied to the adsorbent 3 passes (gaps) between the adsorbents 3 and flows out of the column 2 via the filter member 5, the lid 27, and the outlet pipe 25. At this time, the isolation material (substance to be adsorbed) and the contamination substances other than the isolation material, contained in the sample liquid (sample), are separated from each other based on a difference in adsorbability to the adsorbent 3 and a difference in affinity for the eluate.

In other words, in a state in which the cover portion 22 is located vertically above the cover portion 23, the adsorption apparatus 1 supplies the liquid to the adsorbent filling space 20 via the inlet pipe 24, adsorbs the isolation material by the adsorbent 3 charged in the adsorbent filling space 20, and uses the adsorption of this isolation material to separate the isolation material.

Each of the filter members 4 and 5 has a function of preventing the adsorbent 3 from flowing out of the adsorbent filling space 20, that is, a function of retaining the adsorbent 3 within the adsorbent filling space 20. These filter members 4 and 5 are each composed of, for example, a polypropylene mesh, a sintered filter of polyethylene particles, a stainless mesh filter, or a sintered filter of stainless particles.

In the adsorption apparatus 1, the adsorbent 3 is made of sintered powder, which is obtained by sintering an unbaked powder (unsintered powder) including hydroxyapatite primary particles and secondary particles, as powder formed from a mass of fine particles having a non-constant particle size, and thus has adsorption capability to the isolation material (substance to be adsorbed) contained in the sample liquid (sample).

Herein, the unbaked powder (unsintered powder) including hydroxyapatite primary particles and secondary particles is called "hydroxyapatite powder (or simply called "powder (unsintered powder)"), and the baked hydroxyapatite powder is called "sintered hydroxyapatite powder" (or simply called "sintered powder").

The sintered hydroxyapatite powder is charged in the adsorbent filling space 20 of the column main body 21 as the adsorbent 3. In the present invention, the sintered hydroxyapatite powder is obtained by baking the hydroxyapatite powder at a sintering temperature (baking temperature) of 480°C or higher and 645°C or lower. As a result, the sintered hydroxyapatite powder, i.e., the adsorbent 3 has improved durability and improved adsorbability of a substance to be adsorbed.

The sintered hydroxyapatite powder (sintered powder) as the adsorbent 3 will be described in detail below.

The sintered hydroxyapatite powder is, as described above, obtained by baking the hydroxyapatite powder at a sintering temperature of 480°C or higher and 645°C or lower. The hydroxyapatite powder (powder) is porous bodies with fine pores which contains secondary particles thereof, further primary particles and multi-order particles, and is composed mainly of secondary particles thereof.

The secondary particles of hydroxyapatite (Ca₁₀(PO₄)₆(OH)₂), that is, hydroxyapatite powder, are porous bodies mainly composed of hydroxyapatite, which are obtained by drying a slurry containing the primary particles and their aggregates so as to granulate them. Hydroxyapatite has a chemically stable apatite structure. It is intended that a ratio Ca/P of the hydroxyapatite is about 1.64 or more and 1.70 or less.

The hydroxyapatite secondary particle may be constituted by a secondary particle of fluoroapatite [Ca₁₀(PO₄)₆(OH)₂₋₂ₓF₂ₓ, wherein x is 0 < x ≤ 1] formed by replacing at least part of a hydroxyl group on its surface with fluorine atom. Herein, hydroxyapatite (Ca₁₀(PO₄)₆(OH)₂) and fluoroapatite [Ca₁₀(PO₄)₆(OH)₂₋₂ₓF₂ₓ, wherein x is 0 < x ≤ 1] formed by replacing at least part of its hydroxyl group with fluorine atom are collectively called "hydroxyapatite" as described above.

In the present invention, the hydroxyapatite secondary particles, i.e., the hydroxyapatite powder is obtained by baking the hydroxyapatite powder at a sintering temperature of 480°C or higher and 645°C or lower. The resultant sintered hydroxyapatite powder, i.e., the adsorbent 3 which is composed of porous bodies with fine pores has improved durability and improved adsorbability of a substance to be adsorbed, and specifically has the following configuration.

That is, an average particle compressive strength of the sintered powder (adsorbent 3) satisfies the range of 7.4 MPa or more and 8.9 MPa or less, and a modal pore size in the pore size distribution of the pores of the sintered powder satisfies the range of 32.0 nm or more and 60.0 nm or less.

When the average particle compressive strength and the modal pore size of the sintered powder are within the above ranges, the average particle size of the sintered powder is set within the range of 10 µm or more and 90 µm or less, preferably 20 µm or more and 80 µm or less, and more preferably 40 µm or more and 70 µm or less. When the average particle size of the sintered powder is within the above ranges, and when the average particle compressive strength and modal pore size are set within the above range, the effect obtained by setting the average particle compressive strength and modal pore size within the above ranges can be more prominently achieved.

The average particle compressive strength of the sintered powder may be 7.4 MPa or more and 8.9 MPa or less, but is preferably 8.0 MPa or more and 8.9 MPa or less, and more preferably 8.2 MPa or more and 8.7 MPa or less. In other words, a lower limit value of the average particle compressive strength of the sintered powder may be 7.4 MPa or more, but is preferably 8.0 MPa or more, and more preferably 8.2 MPa or more, and its upper limit value may be 8.9 MPa or less, but is preferably 8.9 MPa or less, and more preferably 8.7 MPa or less. As a result, is can be said that the sintered powder, i.e., the adsorbent 3 has more improved durability.

The modal pore size in the pore size distribution of the pores of the sintered powder (porous bodies) may be 32.0 nm or more and 60.0 nm or less, but is preferably 35.0 nm or more and 60.0 nm or less, and more preferably 42.0 nm or more and 58.0 nm or less. In other words, a lower limit value of the modal pore size may be 32.0 nm or more, but is preferably 35.0 nm or more, and more preferably 42.0 nm or more, and its upper limit value may be 60.0 nm or less, but is preferably 60.0 nm or less, and more preferably 58.0 nm or less. By setting the modal pore size within the above range, even relatively large proteins such as antibodies (antibody molecules) can be reliably adsorbed to the sintered powder by capturing them in the pores of the sintered powder as the isolation material. Therefore, it can be said that the sintered powder, i.e., the adsorbent 3 has more improved adsorbability of the isolation material (substance to be adsorbed).

A frequency of the modal pore size in the pore size distribution of the pores of the sintered powder (porous bodies) is preferably 21.0% or more and 60.0% or less, and more preferably 25.0% or more and 55.0% or less. In other words, a lower limit value of the frequency of the modal pore size is preferably 21.0% or more, and more preferably 25.0% or more, and its upper limit value is preferably 60.0% or less, and more preferably 55.0% or less.

The half-width of the mountain-shaped waveform around the modal pore size (half-width of the modal pore size) is 1.0 nm or more and 10.0 nm or less, more preferably 2.0 nm or more and 10.0 nm or less, further preferably 3.0 nm or more and 10.0 nm or less, and particularly preferably 5.0 nm or more and 10.0 nm or less. In other words, a lower limit value of the half-width is preferably 1.0 nm or more, and more preferably 2.0 nm or more, further preferably 3.0 nm or more, and particularly preferably 5.0 nm or more, and its upper limit value is preferably 10.0 nm or less.

By setting various values in the pore size distribution of the pores of the sintered powder (porous bodies) within the above ranges, the effect obtained by setting the modal pore size within the above range can be more prominently achieved.

An average pore size of the pores of the sintered powder is preferably 37.0 nm or more and 75.0 nm or less, and more preferably 41.0 nm or more and 58.0 nm or less. In other words, a lower limit value of the average pore size is preferably 37.0 nm or more, and more preferably 41.0 nm or more, and its upper limit value is preferably 75.0 nm or less, and more preferably 58.0 nm or less. As a result, the effect obtained by setting the modal pore size within the above range can be more prominently achieved.

The specific surface area of the sintered powder may be 41 m²/g or more and 51 m²/g or less, and is preferably 41 m²/g or more and 49 m²/g or less, and more preferably 41 m²/g or more and 47 m²/g or less. In other words, a lower limit value of the specific surface area may be 41 m²/g or more, and its upper limit value may be 51 m²/g or less, and is preferably 49 m²/g or less, and more preferably 47 m²/g or less. In addition, the surface area of the sintered powder per 1 mL of the column may be 25.3 m²/mL or more and 36.0 m²/mL or less, but is preferably 25.3 m²/mL or more and 34.0 m²/mL or less, and more preferably 25.3 m²/mL or more and 33.0 m²/mL or less. In other words, the surface area may have a lower limit value of 5.3 m²/mL or more and an upper limit value of 36.0 m²/mL or less, but the upper limit value is preferably 34.0 m²/mL or less, and more preferably 33.0 m²/mL or less. By setting the specific surface area of the sintered powder and the surface area of the sintered powder per 1 mL of the column within the above ranges, respectively, even if the isolation material (substance to be adsorbed) is a relatively large protein such as antibodies (antibody molecules), it can be said that sintered powder has the surface area necessary to adsorb a sufficient amount of the isolation material. Therefore, the sintered powder, i.e., the adsorbent 3 can have improved adsorbability of the isolation material (substance to be adsorbed).

The hydroxyapatite powder used in the calcination to obtain sintered hydroxyapatite powder, i.e., the secondary particles of hydroxyapatite, is not particularly limited, but its bulk density is preferably set to 0.65 g/mL or more, and more preferably approximately 0.70 g/mL or more and 0.95 g/mL or less. In other words, a lower limit value of the bulk density is preferably 0.65 g/mL or more, and more preferably 0.70 g/mL or more, and its upper limit value is more preferably 0.95 g/mL or less. Secondary particles having a bulk density within such a range are considered to have a high weight and less voids in the particles, and can be said to be particles having a high filling density, and therefore exhibit high strength. Therefore, the hydroxyapatite powder obtained by baking the hydroxyapatite powder containing the secondary particles also has high strength, so the average particle compressive strength of the sintered powder can be set relatively easily within the above range.

The specific surface area of hydroxyapatite powder (hydroxyapatite secondary particles) is preferably set to 70 m²/g or more, and more preferably approximately 75 m²/g or more and 100 m²/g or less. In other words, a lower limit value of the specific surface area is preferably 70 m²/g or more, and more preferably 75 m²/g or more, and its upper limit value is more preferably approximately 100 m²/g or less. By baking the hydroxyapatite powder having a high specific surface area within such a range, the specific surface area of the resultant sintered hydroxyapatite powder can be set relatively easily within the above range.

Furthermore, a form (shape) of the hydroxyapatite powder is preferably particulate (granular), but a sphericity thereof is more preferably approximately 0.95 or greater and 1.00 or less, and further preferably 0.97 or greater and 1.00 or less. In other words, a lower limit value of the sphericity is preferably 0.95 or more, and more preferably 0.97 or more, and its upper limit value is preferably 1.00or less. In this manner, when the sintered powder derived from the hydroxyapatite powder having high sphericity is applied as the adsorbent 3, it is possible to improve a filling rate of the adsorbent 3 into the adsorbent filling space 20.

As described above, in the present invention, the sintered powder (adsorbent 3) satisfies that the average particle compressive strength is 7.4 MPa or more and 8.9 MPa or less, and that the modal pore size in the pore size distribution of the pores of the sintered powder is 32.0 nm or more and 60.0 nm or less, thereby having improved durability and improved adsorbability of the substance to be adsorbed. Specifically, the durability and adsorbability exhibit the following characteristics.

In other words, when the durability test shown below is carried out on the sintered hydroxyapatite powder, the durability test can be carried out for 60 or more cycles, so it can be said that the sintered hydroxyapatite powder has improved durability.

Specifically, this durability test comprises preparing a column (ϕ4.0×100 mm) which is dry-packed with the sintered hydroxyapatite powder as the adsorbent, and sending each buffer through this column in the order of step numbers shown in Table 2 below. Steps 1 to 5 in Table 2 below are counted as one cycle, and the cycle is repeated until the pressure inside the column exceeds 200 psi. When this durability test is performed on the sintered hydroxyapatite powder (adsorbent of the present invention), the number of cycles repeated can be 60 or more.

**[Table 2]**

| Step No. | Buffer Type | | Supply Amount (CV) | (mL) | Supply Time (hr) |
|---|---|---|---|---|---|
| 1 | 10 mM NaP pH 6.5 | | 15 | 18.8 | 0.63 |
| 2 | 10 mM NaP + 1 M NaCl | pH 6.5 | 10 | 12.6 | 0.42 |
| 3 | 0.4 M NaP pH 6.5 | | 10 | 12.6 | 0.42 |
| 4 | 3 M NaOH | | 10 | 12.6 | 0.42 |
| 5 | 0.4 M NaP pH 6.5 | | 10 | 12.6 | 0.42 |
| | SUM | | 55 | 69.1 | 2.30 |
| Flow rate (mL/min) | | 0.50 | | | |
| Linear velocity (cm/hr) | | 239 | | | |

As described above, the sintered hydroxyapatite powder has improved durability because it can perform 60 or more cycles in the durability test. The number of the cycles may be 60 or more, but is preferably 80 or more, and more preferably 100 or more, which indicates that the sintered hydroxyapatite powder has improved durability.

In addition, when the antibody adsorption amount measurement test shown below was carried out, the sintered hydroxyapatite powder satisfied the following relationship, and the adsorption amount of antibodies as a substance to be adsorbed by the sintered hydroxyapatite powder was independent of the flow rate (flow amount) supplied into the column of the test liquid containing antibodies, so it can be said that the sintered hydroxyapatite powder has improved adsorbability of the substance to be adsorbed.

Specifically, in this antibody adsorption amount measurement test, a column (ϕ6.0×35 mm) is prepared in which the sintered hydroxyapatite powder is dry-packed as an adsorbent, and a sample liquid is prepared by dissolving γ-Globulins from bovine blood, as the substance to be adsorbed, in 10 mM sodium phosphate buffer (pH 6.5) so that the concentration is 1.0 mg/mL. Then, the prepared sample liquid was supplied into this column at flow rates of 150 cm/h, 200 cm/h, 250 cm/h, and 300 cm/h. For each flow rate, the absorbance value X (280 nm) of the sample liquid to be supplied into the column and the absorbance value Y (wavelength: 280 nm) of the eluate eluted from the column are measured, and the adsorption amounts A (mg/mL), B (mg/mL), C (mg/mL), and D (mg/mL) of the substance to be adsorbed to the sintered hydroxyapatite powder are calculated based on the amount of the sample liquid sent until the absorbance value Y (wavelength: 280 nm) becomes 10% of the absorbance value X (wavelength: 280 nm). When such an antibody adsorption amount measurement test was performed on the sintered hydroxyapatite powder (adsorbent of the present invention), the relational expressions D/A, D/B, and C/B were determined based on the adsorption amounts A to D (mg/mL) of the substance to be adsorbed calculated in the antibody adsorption amount measurement test. The relational expression D/A satisfies at least one of A≥31 mg/mL or more and 0.60≤D/A≤1.00, and 0.60≤D/A≤0.92, the relational expression D/B satisfies at least one of B≥30 mg/mL or more and 0.75≤D/B≤1.00, and 0.75≤D/B≤0.96, and the relational expression C/B satisfies at least one of B≥30 mg/mL or more and 0.89≤C/B≤1.00, and 0.89≤C/B≤0.96.

In this manner, the sintered hydroxyapatite powder can make the relational expressions D/A, D/B, and C/B calculated in the antibody adsorption amount measurement test satisfy the above relationships, respectively. Thus, it can be said that the adsorption amount of antibodies (substances to be adsorbed) to the sintered hydroxyapatite powder is independent of the flow rate (flow amount) supplied into the column of the test liquid containing the antibodies. Therefore, during isolation and purification of antibodies using the adsorption apparatus 1 including the sintered hydroxyapatite powder as the adsorbent 3, for example, when supplying the sample liquid containing antibodies to the adsorption apparatus 1, even if the sample liquid cannot be supplied at a constant rate and thus non-uniformity occurs in the initial and final stages, the antibodies can be reliably adsorbed onto the sintered hydroxyapatite powder at a constant range of adsorption amount.

The relational expression D/A may satisfy at least one of A≥31 mg/mL or more and 0.60≤D/A≤1.00, and 0.60≤D/A≤0.92, but preferably satisfies 0.61≤D/A≤0.88. The relational expression D/B may satisfy at least one of B≥30 mg/mL or more and 0.75≤D/B≤1.00, and 0.75≤D/B≤0.96, but preferably satisfies 0.76≤D/B≤0.90. The relational expression C/B satisfies at least one of B≥30 mg/mL or more and 0.89≤CB≤1.00, and 0.89≤C/B≤0.96, but preferably satisfies 0.89≤C/B≤0.95. It can be said that these allow the sintered hydroxyapatite powder to have improved adsorbability of the substance to be adsorbed.

In this antibody adsorption amount measurement test, the adsorption amounts A to D of the substance to be adsorbed are preferably approximately 30 to 31 mg/mL or more, more preferably approximately 34 mg/mL or more, and further preferably approximately 37 mg/mL or more and 45 mg/mL or less. In other words, a lower limit value of each of the adsorption amounts A to D is preferably approximately 30 to 31 mg/mL or more, more preferably approximately 34 mg/mL or more, and further preferably approximately 37 mg/mL, and its upper limit value is further preferably approximately 45 mg/mL or less. As a result, even if the isolation material (substance to be adsorbed) to be isolated using the adsorbent 3 is relatively large proteins such as antibodies (antibody molecules), it can be said that a sufficient amount of isolation material can be adsorbed by the adsorbent 3.

### <Method of manufacturing sintered hydroxyapatite powder>

The above sintered hydroxyapatite powder, i.e., the adsorbent 3, is manufactured, for example, by the following adsorbent manufacturing method.

Note that a description is given below as an example of a case in which as a hydroxyapatite powder to obtain the sintered hydroxyapatite powder, the hydroxyapatite powder with a bulk density of 0.65 g/mL or more and a specific surface area of 70 m²/g or more is prepared.

In this embodiment, the method for manufacturing powder includes a step [S1A] of reacting a first liquid containing a calcium source such as calcium hydroxide and a second liquid containing a phosphoric source such as phosphoric acid while stirring to obtain a slurry containing primary particles of hydroxyapatite and aggregates thereof; a step [S2A] of physically pulverizing the aggregates contained in the slurry and dispersing the pulverized aggregates in the slurry; a step [S3A] of drying the slurry to granulate the pulverized aggregates, thereby obtaining a hydroxyapatite powder composed of particles mainly containing secondary particles of hydroxyapatite; and a step [S4A] of baking the hydroxyapatite powder to obtain a sintered hydroxyapatite powder.

These steps will be described sequentially below.

Note that a description is given below as an example of a case in which calcium hydroxide is used as the calcium source, and phosphoric acid is used as the phosphoric source.

### [S1A: Step of forming slurry containing hydroxyapatite aggregates]

In this step, by stirring a calcium hydroxide dispersion (first liquid) containing calcium hydroxide and a phosphoric acid solution (second liquid) containing phosphoric acid, calcium hydroxide are reacted with phosphoric acid to form a slurry containing hydroxyapatite primary particles and their aggregates. That is, a slurry containing hydroxyapatite primary particles and their aggregates is prepared.

Specifically, for example, a slurry containing hydroxyapatite primary particles and their aggregates is obtained by dropping a phosphoric acid solution (second liquid) into a calcium hydroxide dispersion (first liquid) while being stirred in a container (not shown) to form a mixed liquid of the calcium hydroxide dispersion and the phosphoric acid solution, so that calcium hydroxide are reacted with phosphoric acid in the mixed liquid.

This method employs a wet synthesis method using a phosphoric acid solution containing phosphoric acid, which does not need an expensive manufacture equipment and can synthesize hydroxyapatite (compound) more easily and effectively. In addition, this reaction has an advantage in that any by-products do not remain in the resultant secondary particles and sintered powder because the reaction between calcium hydroxide and phosphoric acid generates only water as a by-product. Furthermore, because this reaction is an acid-base reaction, this reaction can be easily controlled by adjusting the pHs of the calcium hydroxide dispersion and the phosphoric acid solution.

The phosphoric acid solution containing phosphoric acid may be a phosphoric acid aqueous solution, in which a small amount of other liquid such as alcohol may be added.

Stirring can effectively promote the reaction between calcium hydroxide and phosphoric acid, that is, can enhance the efficiency of the reaction.

The force of stirring the mixed liquid containing the calcium hydroxide dispersion and the phosphoric acid solution is not particularly limited, but is preferably approximately 0.75 W or more and 2.0 W or less, and more preferably approximately 0.925 W or more and 1.85 W or less, per 1 L of the mixed liquid (slurry). In other words, a lower limit value of the stirring force is preferably 0.75 W or more, and more preferably 0.925 W or more, and its upper limit value is preferably 2.0 W or less, and more preferably 1.85 W or less, per 1 L of the mixed liquid (slurry). By setting the stirring force within this range, the efficiency of the reaction between calcium hydroxide and phosphoric acid can be further improved.

The content of calcium hydroxide in the calcium hydroxide dispersion is preferably approximately 5% by mass or more and 15% by mass or less, and more preferably approximately 10% by mass or more and 12% by mass or less. In other words, a lower limit value of the content of calcium hydroxide is preferably 5% by mass or more, and more preferably 10% by mass or more, and its upper limit value is preferably 15% by mass or less, and more preferably 12% by mass or less. The content of phosphoric acid in the phosphoric acid solution is preferably approximately 10% by mass or more and 25% by mass or less, and more preferably approximately 15% by mass or more and 20% by mass or less. In other words, a lower limit value of the content of phosphoric acid is preferably 10% by mass or more, and more preferably 15% by mass or more, and its upper limit value is preferably 25% by mass or less, and more preferably 20% by mass or less. By setting the contents of calcium hydroxide and phosphoric acid in these ranges, the contact chances between calcium hydroxide and phosphoric acid increase when the phosphoric acid solution are dropped into the calcium hydroxide dispersion while being stirred, and thus calcium hydroxide can be effectively reacted with phosphoric acid, thereby reliably synthesizing hydroxyapatite.

The dropping rate of the phosphoric acid solution is preferably approximately 1 L/hr or more and 40 L/hr or less, and more preferably approximately 3 L/hr or more and 30 L/hr or less. In other words, a lower limit value of the dropping rate of the phosphoric acid solution is preferably 1 L/hr or more, and more preferably 3 L/hr or more, and its upper limit value is preferably 40 L/hr or less, and more preferably 30 L/hr or less. By mixing (adding) the phosphoric acid solution in the calcium hydroxide dispersion at this dropping rate, calcium hydroxide can be reacted with phosphoric acid under milder conditions.

In this case, the dropping time (adding time) of the phosphoric acid solution is preferably approximately 5 hours or more and 32 hours or less, and more preferably approximately 6 hours or more and 30 hours or less. In other words, a lower limit value of the dropping time of the phosphoric acid solution is preferably 5 hours or more, and more preferably 6 hours or more, and its upper limit value is preferably 32 hours or less, and more preferably 30 hours or less. By reacting calcium hydroxide with phosphoric acid for this dropping time, hydroxyapatite can be sufficiently synthesized. It is noted that even if the dropping time is made longer than the above upper limit, further progress of the reaction between calcium hydroxide and phosphoric acid cannot be expected.

When the reaction between calcium hydroxide and phosphoric acid gradually proceeds, fine particles of hydroxyapatite (compound) (hereinafter, simply called "fine particles") are generated in the slurry. The fine particles then aggregate by van der Waals force (intermolecular force) generated between the positively charged portion of one fine particle (primary particle) and the negatively charged portion of the other fine particle, so as to form aggregates of hydroxyapatite (compound) (hereinafter, simply called "aggregates"). As the aggregates are formed, the viscosity of the slurry gradually increases.

Furthermore, as the reaction between calcium hydroxide and phosphoric acid proceeds, a ratio between positive charges and negative charges in the slurry becomes closer to even. At this time, a repulsive force applied to the fine particles decreases in the slurry, thereby accelerating the aggregation of the fine particles, resulting in formation of aggregates with larger particle size.

### [S2A: Step of pulverizing and dispersing aggregates]

In this step, the aggregates of primary particles of hydroxyapatite contained in the slurry obtained in step [S1A] are physically pulverized, and the pulverized aggregates are dispersed in the slurry.

By crushing the aggregates contained in the slurry as described above, the particle size of the aggregates contained in the slurry becomes smaller, and as a result, the secondary particles of hydroxyapatite obtained in the subsequent step [S3A] can be set to have a bulk density of 0.65 g/mL or more and a specific surface area of 70 m²/g or more.

The method for physically pulverizing aggregates of primary particles of hydroxyapatite is not particularly limited, but includes, for example, a wet jet milling method, in which droplets of slurry sprayed at high pressure are collided with each other, and a ball mill method, in which the slurry is placed in a closed vessel in the presence of spheres made of ceramics such as zirconia and the closed vessel is rotated. Among them, the wet jet milling method is preferable.

The wet jet milling method is a method in which high pressure is applied to a slurry in which aggregates of primary particles of hydroxyapatite are dispersed, and the slurry is sprayed into droplets, which are then introduced into an opposed collision chamber, a ball collision chamber, or a single nozzle chamber, where the aggregates are pulverized by the collision between them.

According to this method, aggregates of primary particles of hydroxyapatite can be reliably pulverized. Therefore, the secondary particles of hydroxyapatite obtained in the subsequent step [S3A] can be set to have a bulk density of 0.65 g/mL or more.

Moreover, the average particle size of the pulverized aggregates is preferably 1 µm or less, and more preferably 0.1 µm or more and 0.6 µm or less. By controlling the average particle size of the pulverized aggregates within this range, the bulk density and specific surface area of the secondary particles of hydroxyapatite obtained in the subsequent step [S3A] can be set within the above ranges.

As in the present embodiment, when the secondary particles of hydroxyapatite obtained in the subsequent step [S3A] are not set to have a bulk density of 0.65 g/mL or more and a specific surface area of 70 m²/g or more, the physical pulverization of the aggregates in this step [S2A] can be omitted. However, by physically pulverizing the aggregates to set the bulk density of the secondary particles to 0.65 g/mL or more and the specific surface area to 70 m²/g or more, when the hydroxyapatite powder is baked at a sintering temperature of approximately 480°C or higher and 645°C or lower in the subsequent step [S4A], the resultant sintered hydroxyapatite powder can reliably have both improved durability and improved adsorbability of the substance to be adsorbed. In other words, the sintered hydroxyapatite powder can satisfy an average particle compressive strength of 7.4 MPa or more and 8.9 MPa or less, and a modal pore size of 32.0 nm or more and 60.0 nm or less.

### [S3A: Step of drying slurry to obtain hydroxyapatite powder]

In this step, the slurry which is prepared in step [S2A] and contains pulverized aggregates is dried so as to granulate the pulverized aggregates into unsintered powder (dried powder) mainly composed of hydroxyapatite secondary particles, that is, hydroxyapatite powder.

In the present embodiment, in step [S2A], the aggregates of primary particles of hydroxyapatite are pulverized, and thus the size of the aggregates is small, which can provide the hydroxyapatite powder obtained in step [S3A] with a bulk density of 0.65 g/mL or more, and a specific surface area of 70 m²/g or more.

The method of drying the slurry is not particularly limited, but is preferably a spray-drying method. According to this method, pulverized aggregates are granulated so that powder with a desired particle size can be obtained more reliably and in a shorter time.

The drying temperature of the slurry is preferably about 75°C or higher and 250°C or lower, and more preferably about 95°C or higher and 220°C or lower. In other words, a lower limit value of the drying temperature is preferably 75°C or higher, and more preferably 95°C or higher, and its upper limit value is preferably 250°C or lower, and more preferably 220°C or lower. By setting the drying temperature in this range, secondary particles with a high bulk density and a large specific surface area can be obtained.

The above steps [S1A] to [S3A] constitute a first step of preparing hydroxyapatite powder.

Note that, unlike the present embodiment, when the hydroxyapatite secondary particles obtained in the subsequent step [S3A] are not set to have a bulk density of 0.65 g/mL or more and a specific surface area of 70 m²/g or more, the hydroxyapatite powder does not need to be adjusted by the above steps [S1A] to [S3A], and may be prepared, for example, according to the method described in the specification of JP 7-88205 A.

### [S4A: Step of baking hydroxyapatite powder to obtain sintered hydroxyapatite powder]

In this step, the hydroxyapatite powder produced in the above step [S3A] is sintered by baking to produce a sintered powder composed of hydroxyapatite secondary particles, i.e., sintered hydroxyapatite powder (second step).

In this case, the sintering temperature for baking the powder may be set to approximately 480°C or higher and 645°C or lower, but preferably approximately 500°C or higher and 630°C or lower, and more preferably 550°C or higher and 610°C or lower. In other words, a lower limit value of the sintering temperature may be 480°C or higher, but is preferably 500°C or higher, and more preferably 550°C or higher, and its upper limit value may be 645°C or lower, but is preferably 630°C or lower, and more preferably 610°C or lower.

The time of holding the sintering temperature for baking the powder is not particularly limited, but is preferably about 60 minutes or more and 230 minutes or less, and more preferably about 90 minutes or more and 180 minutes or less. In other words, a lower limit value of the holding time is preferably 60 minutes or more, and more preferably 90 minutes or more, and its upper limit value is preferably 230 minutes or less, and more preferably 180 minutes or less.

By setting the conditions for baking the powder within the above ranges, the resultant sintered hydroxyapatite powder (adsorbent 3) can be relatively easily made to have an average particle compressive strength of 7.4 MPa or more and 8.9 MPa or less, and a modal pore size in the pore size distribution of the pores of the sintered powder of 32.0 nm or more and 60.0 nm or less.

Through the above steps, sintered powder composed of sintered particles mainly containing hydroxyapatite secondary particles, i.e., sintered hydroxyapatite powder, is obtained.

By filling this sintered hydroxyapatite powder in the adsorbent filling space 20 of the column 2 as the adsorbent 3, the adsorption apparatus 1 can be obtained. When the sintered hydroxyapatite powder to be filled is classified, the sintered hydroxyapatite powder may have, for example, the average particle size and particle size distribution of 20 ± 4 µm, 40 ± 4 µm, 60 ± 4 µm, 80 ± 4 µm, etc.

Although the adsorbent of the present invention and its production method are described above, the present invention is not restricted thereto.

For example, each component of the adsorbent of the present invention may be replaced with whatever exhibits the same function, and the adsorbent of the present invention may be added with arbitrary components. In addition, the method of producing the adsorbent of the present invention may be added with one or more steps for any purpose.

### Examples

Next, specific examples of the present invention will be described.

Note that the present invention is not limited to the description of these examples.

### 1. Manufacture of sintered hydroxyapatite powder

### (Example 1)

[1A] 2400 g calcium hydroxide was dispersed in 60 L pure water, and the resultant calcium hydroxide dispersion was supplied in a tank. With the calcium hydroxide dispersion stirred, 4 L phosphoric acid aqueous solution (phosphoric acid concentration: 85% by mass) was dropped therein at a rate of 1 L/hr, obtaining a slurry containing aggregates in which 10% by mass of hydroxyapatite primary particles were aggregated.

The atmosphere temperature during dropping was a room temperature (25°C).

The force of stirring the mixed liquid, in which the phosphoric acid aqueous solution was dropped in the dispersion, was 1.7 W per 1L of the mixed liquid (slurry).

[2A] The aggregates contained in the obtained slurry were pulverized using a wet jet milling device ("Star Burst" manufactured by Sugino Machine Limited.) at a high pressure of 245 MPa to obtain a slurry containing pulverized aggregates with an average particle size of 1.3 µm.

The average particle size of the aggregates contained in the slurry after pulverization was measured using a particle size distribution analyzer ("MT3300" manufactured by Microtrac Inc.).

[3A] The slurry containing the pulverized (dispersed) aggregates was spray-dried at 210°C using a spray drier ("MAD-6737R" manufactured by MATSUBO Corporation) to granulate hydroxyapatite contained in the slurry, thereby obtaining spherical secondary particles (dried powder). The resultant dried powder, i.e., hydroxyapatite powder, was classified using an ultrasonic sieve to obtain hydroxyapatite powder with an average particle size of approximately 40 µm, more specifically, hydroxyapatite powder with a particle size of 40 µm ± 4.0 µm.

The powder (secondary particles) was confirmed to be hydroxyapatite by a powder X-ray diffractometry.

[4A] The classified hydroxyapatite powder was baked at a sintering temperature of 500°C to obtain the sintered hydroxyapatite powder of Example 1.

### (Example 2)

The sintered hydroxyapatite powder of Example 2 was obtained in the same manner as in Example 1, except that, in step [4A], the sintering temperature for baking the hydroxyapatite powder was changed to 600°C.

### (Comparative Example 1)

The sintered hydroxyapatite powder of Comparative Example 1 was obtained in the same manner as in Example 1, except that, in step [4A], the sintering temperature for baking the hydroxyapatite powder was changed to 400°C.

### (Comparative Example 2)

The sintered hydroxyapatite powder of Comparative Example 2 was obtained in the same manner as in Example 1, except that, in step [4A], the sintering temperature for baking the hydroxyapatite powder was changed to 700°C.

### 2. Evaluation of sintered hydroxyapatite powder

### 2-1. Evaluation of average particle compressive strength

The average particle compressive strength of the sintered hydroxyapatite powder of each Example and Comparative Example was determined using a micro compression testing machine ("MCT-W200-J" manufactured by Shimadzu Corporation).

The average particle compressive strength of the sintered hydroxyapatite powder of each Example and Comparative Example was obtained by measuring the particle compressive strength 10 times and calculating the average value. The calculating results are shown in Table 3.

**[Table 3]**

| | Sintering Temperature (°C) | Average Particle Compressive Strength (MPa) |
|---|---|---|
| Comparative Example 1 | 400 | 6.180 |
| Example 1 | 500 | 7.611 |
| Example 2 | 600 | 8.570 |
| Comparative Example 2 | 700 | 9.827 |

As is clear from Table 3, for the sintered powder of each Example and Comparative Example, by setting the sintering temperature of hydroxyapatite powder high, the sintered hydroxyapatite powder with excellent particle compressive strength was obtained.

### 2-2. Evaluation of pore size

For the sintered hydroxyapatite powders of each Example and Comparative Example, the pore size distribution of the pores on their surfaces was measured by a mercury porosimetry using a pore distribution measuring device ("Micromeritics AutoPore 9200" manufactured by Shimadzu Corporation). From this pore size distribution, the modal pore size, the frequency of the modal pore size, the half-width of the mountain-shaped waveform around the modal pore size (half-width of the modal pore size), and the average pore size were calculated. The calculating results are shown in Table 4.

**[Table 4]**

| | Sintering Temperature (°C) | Modal Pore Size (nm) | Frequency of Modal Pore Size (%) | Half-Width of Modal Pore Size (nm) | Average Pore Size (nm) |
|---|---|---|---|---|---|
| Comparative Example 1 | 400 | 41.4 | 16.0 | 11.0 | 36.8 |
| Example 1 | 500 | 41.4 | 19.2 | 10.2 | 42.6 |
| Example 2 | 600 | 54.2 | 32.7 | 8.6 | 56.5 |
| Comparative Example 2 | 700 | 71.0 | 38.5 | 10.7 | 75.6 |

As is clear from Table 4, for the sintered powder of each Example and Comparative Example, by setting the sintering temperature of hydroxyapatite powder higher, the sintered hydroxyapatite powder with larger pore size was obtained.

### 2-3. Evaluation of specific surface area

The specific surface area (average specific surface area) of the sintered hydroxyapatite powder of each Example and Comparative Example was determined using a fully auto BET specific surface area measuring device ("Macsorb HM1201" manufactured by Mountech Co.,Ltd.). The measuring results are shown in Table 5.

**[Table 5]**

| | Sintering Temperature (°C) | Average Specific Surface Area (m²/g) |
|---|---|---|
| Comparative Example 1 | 400 | 51.66 |
| Example 1 | 500 | 42.41 |
| Example 2 | 600 | 29.69 |
| Comparative Example 2 | 700 | 20.75 |

As is clear from Table 5, for the sintered powder of each Example and Comparative Example, by setting the sintering temperature of hydroxyapatite powder higher, the specific surface area tends to become smaller.

### 2-4. Evaluation of the surface area of sintered powder per 1 mL of column

From the data obtained from the evaluation of the specific surface area, the surface area per 1 mL of column in the sintered powder of each Example and Comparative Example was calculated. The measuring results are shown in Table 6.

**[Table 6]**

| | Sintering Temperature (°C) | Surface Area per 1 mL of Column (m²/mL) |
|---|---|---|
| Comparative Example 1 | 400 | 35.51 |
| Example 1 | 500 | 29.37 |
| Example 2 | 600 | 20.91 |
| Comparative Example 2 | 700 | 14.73 |

As is clear from Table 6, for the sintered powder of each Example and Comparative Example, by setting the sintering temperature of hydroxyapatite powder higher, the surface area per 1 mL of column, as well as the specific surface area, tended to become smaller.

### 2-5. Evaluation of antibody adsorption amount

For columns equipped with the sintered hydroxyapatite powder of each Example and Comparative Example as the adsorbent, sample liquid containing antibodies was supplied into the columns at flow rates of 150, 200, 250, and 300 cm/hr, respectively, and the antibody adsorption amount to the adsorbent was measured using the method shown below.

First, γ-Globulins from bovine blood (bIgG) were dissolved in 10 mM NaP (sodium phosphate buffer) at pH 6.5 to a concentration of 1.0 mg/mL to prepare a sample liquid (bIgG solution).

Next, columns (ϕ6.0×35 mm) were prepared in which the sintered hydroxyapatite powder of each Example and Comparative Example was dry-packed as an adsorbent, and the prepared sample liquid was sent from a chromatographic pump and supplied into the columns at flow rates of 150, 200, 250, and 300 cm/hr, respectively. Then, the absorbance value X (wavelength: 280 nm) of the sample liquid to be supplied into the column and the absorbance value Y(wavelength: 280 nm) of the eluate eluted from the column were measured, and the sample liquid was sent until the absorbance value Y (wavelength: 280 nm) reached 10% of the absorbance value X (wavelength: 280 nm).

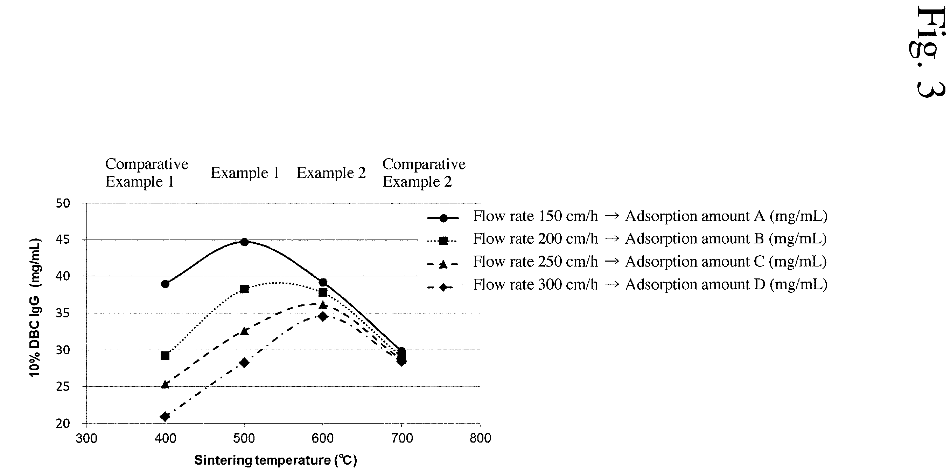

Based on the amount of the sample liquid, the adsorption amounts A to D (mg/mL) of bIgG to the sintered hydroxyapatite powder of each Example and Comparative Example were calculated. Based on the calculated adsorption amounts A to D (mg/mL), the adsorption amount ratios D/A, D/B, and C/B were also calculated. The calculating results are shown in Table 7 and Fig.3.

**[Table7]**

| | Sintering Temperature (°C) | Antibody Adsorption Amount at Each Flow Rate (mg/mL) | | | | Relational Expressions based on Antibody Adsorption Amounts | | |
|---|---|---|---|---|---|---|---|---|
| | | A^{*1} | B^{*2} | C^{*3} | D^{*4} | D/A | D/B | C/B |
| Comparative Example 1 | 400 | 38 | 29 | 26 | 22 | 0.58 | 0.76 | 0.90 |
| Example 1 | 500 | 45 | 37 | 33 | 28 | 0.62 | 0.76 | 0.89 |
| Example 2 | 600 | 39 | 38 | 36 | 34 | 0.87 | 0.89 | 0.95 |
| Comparative Example 2 | 700 | 30 | 29 | 28 | 28 | 0.93 | 0.97 | 0.97 |

| | | | | | | | | |
|---|---|---|---|---|---|---|---|---|
| Note *1: Adsorption amount at a flow rate of 150 cm/hr Note *2: Adsorption amount at a flow rate of 200 cm/hr Note *3: Adsorption amount at a flow rate of 250 cm/hr Note *4: Adsorption amount at a flow rate of 300 cm/hr | | | | | | | | |

As is clear from Table 7, the sintered powders of each Example, compared with the sintered powders of each Comparative Example, were independent of the flow rate (flow amount) at which test liquid containing antibodies was supplied to the column.

### 2-6. Evaluation of durability

The durability of the adsorbent in the column of each Example and Comparative Example, which contained the sintered hydroxyapatite powder as the adsorbent, was evaluated using the following method.

First, columns (ϕ4.0×100 mm) were prepared in which the sintered hydroxyapatite powder of each Example and Comparative Example was dry-packed as an adsorbent. Here, dry packing means that the dried sintered hydroxyapatite powder was added into the column and tapped 120 times by hand to pack the sintered powder densely without destroying it.

Next, each buffer was supplied into these columns in the order of step numbers shown in Table 8. Steps 1 to 5 were counted as one cycle, and were repeated until the pressure inside the column exceeded 200 psi. The results of this cycle are shown in Table 9.

**[Table 8]**

| Step No. | Buffer Type | | Supply Amount | | Supply Time |
|---|---|---|---|---|---|
| | | | (CV) | (mL) | (hr) |
| 1 | 10 mM NaP pH 6.5 | | 15 | 18.8 | 0.63 |
| 2 | 10 mM NaP + 1 M NaCl | pH 6.5 | 10 | 12.6 | 0.42 |
| 3 | 0.4 M NaP pH 6.5 | | 10 | 12.6 | 0.42 |
| 4 | 3 M NaOH | | 10 | 12.6 | 0.42 |
| 5 | 0.4 M NaP pH 6.5 | | 10 | 12.6 | 0.42 |
| | SUM | | 55 | 69.1 | 2.30 |
| Flow rate (mL/min) | | 0.50 | | | |
| Linear velocity (cm/hr) | | 239 | | | |

**[Table 9]**

| | Sintering Temperature (°C) | Durability of Adsorbent (cycle) |
|---|---|---|
| Comparative Example 1 | 400 | 50 |
| Example 1 | 500 | 62 |
| Example 2 | 600 | 103 |
| Comparative Example 2 | 700 | 153 |

As is clear from Table 9, the sintered powder of each Example had improved durability compared to the sintered powder of each Comparative Example.

### 2-7. Summary

As described above, in step [4A], by setting the sintering temperature in a process of sintering the hydroxyapatite powder to a temperature in the range of 480°C or higher and 645°C or lower, it has been found that the resultant sintered powder has an average particle compressive strength of 7.4 MPa or more, and the modal pore size in the pore size distribution of the pores of the sintered powder of 32.0 nm or more and 60.0 nm or less.

It has been also revealed that by using a sintered powder whose average particle compressive strength and modal pore size are both within the above-mentioned ranges as the adsorbent 3 in the adsorption apparatus 1, the adsorbent 3 in the adsorption apparatus 1 has improved durability and improved adsorbability of the substance to be adsorbed.

### Reference Signs List

- 1: Adsorption apparatus
- 2: Column
- 3: Adsorbent
- 4: Filter member
- 5: Filter member
- 20: Adsorbent filling space
- 21: Column main body
- 22: Cover portion
- 23: Cover portion
- 24: Inlet pipe
- 25: Outlet pipe
- 26: Lid
- 27: Lid
- 28: Cap
- 29: Cap
- 41: Flow path
- 51: Flow path

## Claims

1. An adsorbent which is to be packed in a column, and is composed of powder containing particles whose surface is made of hydroxyapatite or fluorapatite in which at least some of hydroxyl groups of the hydroxyapatite have been replaced with fluorine atoms,
the powder being composed of porous bodies with pores,
the powder having an average particle size of 10 µm or more and 90 µm or less,
the powder having an average particle compressive strength of 7.4 MPa or more and 8.9 MPa or less, and
a modal pore size in a pore size distribution of the pores of the powder being 32.0 nm or more and 60.0 nm or less.

2. The adsorbent according to claim 1, wherein a specific surface area of the powder is 41 m²/g or more and 51 m²/g or less.

3. The adsorbent according to claim 1, wherein a surface area of the powder per 1 mL of the column is 25.3 m²/mL or more and 36.0 m²/mL or less.

4. An adsorbent which is to be packed in a column, and is composed of sintered powder obtained by baking particles whose surface is made of hydroxyapatite or fluorapatite in which at least some of the hydroxyl groups of the hydroxyapatite have been replaced with fluorine atoms,
the sintered powder having an average particle size of 10 µm or more and 90 µm or less,
the sintered powder having a specific surface area of 41 m²/g or more and 51 m²/g or less, and
a modal pore size in a pore size distribution of pores of the sintered powder being 32.0 nm or more and 60.0 nm or less.

5. An adsorbent which is to be packed in a column, and is composed of powder containing particles whose surface is made of hydroxyapatite or fluorapatite in which at least some of hydroxyl groups of the hydroxyapatite have been replaced with fluorine atoms,
the powder having an average particle size of 10 µm or more and 90 µm or less,
a surface area of the powder per 1 mL of the column being 25.3 m²/mL or more and 36.0 m²/mL or less, and
a modal pore size in a pore size distribution of pores of the powder being 32.0 nm or more and 60.0 nm or less.

6. The adsorbent according to any one of claims 1, 3 and 5, wherein a frequency of the modal pore size is 21.0% or more and 60.0% or less.

7. The adsorbent according to any one of claims 1, 3 and 5, wherein a half-width of a mountain-shaped waveform around the modal pore size in the pore size distribution of the pores is 1.0 nm or more and 10.0 nm or less.

8. The adsorbent according to any one of claims 1, 3 and 5, wherein an average pore size of the pores is 37.0 nm or more and 75.0 nm or less.

9. The adsorbent according to any one of claims 1 to 5, wherein the powder is a sintered powder baked at 480°C or higher and 645°C or lower.

10. A method for manufacturing an adsorbent comprising:
a first step of preparing particles whose surface is made of hydroxyapatite or fluorapatite in which at least some of the hydroxyl groups of the hydroxyapatite have been replaced with fluorine atoms; and
a second step of baking the particles prepared in the first step at a temperature T in the range of 480°C or higher and 645°C or lower to obtain the sintered powder of the particles;
the sintered powder obtained in the second step having an average particle size of 10 µm or more and 90 µm or less, an average particle compressive strength of 7.4 MPa or more and 8.9 MPa or less, and a pore size of 32.0 nm or more and 60.0 nm or less.

11. A method for manufacturing an adsorbent comprising:
a first step of preparing particles whose surface is made of hydroxyapatite or fluorapatite in which at least some of the hydroxyl groups of the hydroxyapatite have been replaced with fluorine atoms; and
a second step of baking the particles prepared in the first step at a temperature T in the range of 480°C or higher and 645°C or lower to obtain the sintered powder of the particles;
the sintered powder obtained in the second step having an average particle size of 10 µm or more and 90 µm or less, a specific surface area of 41 m²/g or more and 51 m²/g or less, and a pore size of 32.0 nm or more and 60.0 nm or less.

12. A method for manufacturing an adsorbent comprising:
a first step of preparing particles whose surface is made of hydroxyapatite or fluorapatite in which at least some of the hydroxyl groups of the hydroxyapatite have been replaced with fluorine atoms; and
a second step of baking the particles prepared in the first step at a temperature T in the range of 480°C or higher and 645°C or lower to obtain the sintered powder of the particles;
the sintered powder obtained in the second step having an average particle size of 10 µm or more and 90 µm or less,
a surface area of the sintered powder per 1 mL of a column when the sintered powder is packed in the column being 25.3 m²/mL or more and 36.0 m²/mL or less, and
a pore size is 32.0 nm or more and 60.0 nm or less.

13. An adsorbent which is to be packed in a column, and is composed of powder containing particles whose surface is made of hydroxyapatite or fluorapatite in which at least some of hydroxyl groups of the hydroxyapatite have been replaced with fluorine atoms, wherein
when a sample liquid is prepared by dissolving γ-Globulins from bovine blood, as a substance to be adsorbed, in 10 mM sodium phosphate buffer (pH 6.5) so that the concentration is 1.0 mg/mL, and the prepared sample liquid is supplied into the column (ϕ6.0×35 mm) at flow rates of 150 cm/h and 300 cm/h, and when for each flow rate, an absorbance value X (280 nm) of the sample liquid to be supplied into the column and an absorbance value Y (wavelength: 280 nm) of an eluate eluted from the column are measured, and adsorption amounts A (mg/mL) and D (mg/mL) of the substance to be adsorbed to the powder are calculated based on the amount of the sample liquid sent until the absorbance value Y (wavelength: 280 nm) becomes 10% of the absorbance value X (wavelength: 280 nm), the powder satisfies at least one of A≥31 mg/mL or more and 0.60≤D/A≤1.00, and 0.60≤D/A≤0.92.

14. An adsorbent which is to be packed in a column, and is composed of powder obtained by baking particles whose surface is made of hydroxyapatite or fluorapatite in which at least some of the hydroxyl groups of the hydroxyapatite have been replaced with fluorine atoms, wherein
when a sample liquid is prepared by dissolving γ-Globulins from bovine blood, as a substance to be adsorbed, in 10 mM sodium phosphate buffer (pH 6.5) so that the concentration is 1.0 mg/mL, and the prepared sample liquid is supplied into the column (ϕ6.0×35 mm) at flow rates of 200 cm/h and 300 cm/h, and when for each flow rate, an absorbance value X (280 nm) of the sample liquid to be supplied into the column and an absorbance value Y (wavelength: 280 nm) of an eluate eluted from the column are measured, and adsorption amounts B (mg/mL) and D (mg/mL) of the substance to be adsorbed to the powder are calculated based on the amount of the sample liquid sent until the absorbance value Y (wavelength: 280 nm) becomes 10% of the absorbance value X (wavelength: 280 nm), the powder satisfies at least one of B≥30 mg/mL or more and 0.75≤D/B≤1.00, and 0.75≤D/B≤0.96.

15. An adsorbent which is to be packed in a column, and is composed of powder obtained by baking particles whose surface is made of hydroxyapatite or fluorapatite in which at least some of the hydroxyl groups of the hydroxyapatite have been replaced with fluorine atoms, wherein
when a sample liquid is prepared by dissolving γ-Globulins from bovine blood, as a substance to be adsorbed, in 10 mM sodium phosphate buffer (pH 6.5) so that the concentration is 1.0 mg/mL, and the prepared sample liquid is supplied into the column (ϕ6.0×35 mm) at flow rates of 200 cm/h and 250 cm/h, and when for each flow rate, an absorbance value X (280 nm) of the sample liquid to be supplied into the column and an absorbance value Y (wavelength: 280 nm) of an eluate eluted from the column are measured, and adsorption amounts B (mg/mL) and C (mg/mL) of the substance to be adsorbed to the powder are calculated based on the amount of the sample liquid sent until the absorbance value Y (wavelength: 280 nm) becomes 10% of the absorbance value X (wavelength: 280 nm), the powder satisfies at least one of B≥30 mg/mL or more and 0.89≤CB≤1.00, and 0.89≤CB≤0.96.

16. The adsorbent according to any one of claims 13 to 15, wherein
when a durability test of the powder is conducted in which the column (ϕ4.0×100 mm) which is dry-packed with the powder as the adsorbent, and each buffer is sent through the column in the order of step numbers shown in Table 1, and regarding that steps 1 to 5 shown in Table 1 are counted as one cycle, the cycle is repeated until the pressure inside the column exceeds 200 psi, the number of cycles repeated is 60 or more.
**[Table 1]**
| Step No. | Buffer Type | | Supply Amount | | Supply Time |
|---|---|---|---|---|---|
| | | | (CV) | (mL) | (hr) |
| 1 | 10 mM NaP pH 6.5 | | 15 | 18.8 | 0.63 |
| 2 | 10 mM NaP + 1 M NaCl pH 6.5 | | 10 | 12.6 | 0.42 |
| 3 | 0.4 M NaP pH 6.5 | | 10 | 12.6 | 0.42 |
| 4 | 3 M NaOH | | 10 | 12.6 | 0.42 |
| 5 | 0.4 M NaP pH 6.5 | | 10 | 12.6 | 0.42 |
| | SUM | | 55 | 69.1 | 2.30 |
| Flow rate (mL/min) | | 0.50 | | | |
| Linear velocity (cm/hr) | | 239 | | | |

17. The adsorbent according to any one of claims 13 to 15, wherein
the substance to be adsorbed is antibodies.
